# EUROPEAN PATENT APPLICATION

(11) **EP 3 841 892 A1**
(43) Date of publication of application: **30.06.2021**
(21) Application number: 20210984.9
(22) Date of filing: 10.05.2017
(51) Int. Cl.: A23L 33/00, A23L 33/19

(54) **FERMENTED INFANT FORMULA**

(30) Priority: 10.05.2016 EP 16169015
(62) Divisional of application: 17728777.8
(71) Applicant: N.V. Nutricia, 2712 HM Zoetermeer (NL)
(72) Inventor: Vereijken, Carolus Maria Johannes Leonardus, 3584 CT Utrecht (NL); Bouritius, Houkje, 3584 CT Utrecht (NL)
(74) Representative: Nederlandsch Octrooibureau

(57) **Abstract**

A fermented infant formula for promoting improved eating behaviour and reducing the risk of obesity.

## Description

### FIELD OF THE INVENTION

The invention relates to nutrition for infants, in particular infant formula.

### BACKGROUND OF THE INVENTION

Human milk is the uncontested gold standard concerning infant nutrition. However, in some cases breastfeeding is inadequate or unsuccessful for medical reasons or because of a choice not to breastfeed. For such situations infant or follow on formulas have been developed. Commercial infant formulas are commonly used today to provide supplemental or sole source of nutrition early in life. These formulas comprise a range of nutrients to meet the nutritional needs of the growing infant, and typically include fat, carbohydrate, protein, vitamins, minerals, and other nutrients helpful for optimal infant growth and development. Commercial infant formulas are designed to mimic, as closely as possible, the composition and function of human milk.

Since long it has been appreciated that breast-fed infants have a different weight gain pattern or trajectory compared to formula-fed infants. Numerous studies performed in various regions from all over the world have reported that breast-fed infants have a slower weight gain. Length gain tends to differ less between breast-fed and formula-fed infants and as a result breast-fed infants are leaner. Thus, in the art it has been indicated that the growth curve of infants fed with commercial infant formula differs from the growth curve of breast-fed infants. Typically an infant formula has a growth (weight) accelerating effect in the first year of life. Furthermore this difference in growth trajectories is correlated with an increased Body Mass Index early in life and an increased risk of developing obesity later in life. Li et al, 2010, Pediatrics 125:e1386-e1393 discloses that infants that are bottle-fed in early infancy are more likely to empty the bottle or cup in late infancy than those who are fed directly at the breast. Bottle-feeding, regardless of the type of milk, is distinct from feeding at the breast in its effect on infants' self-regulation of milk intake.

In the prior art in the field of infant formula for improving the growth trajectory to be more similar to the growth trajectory of breast-fed infants, the focus is on infant formula with lower protein and/or lower caloric density. WO 2008/071667 discloses a nutritional composition for infants at risk of developing obesity later in life comprising a protein source, a lipid source and a carbohydrate source. The protein content is less than 1.8 g/100 kcal and the energy density is less than 650 kcal/litre. In WO 2010/070613 it is disclosed that a lower weight gain in the first week of life was observed when using a formula with a very low caloric content and low protein content based on volume. Koletzko et al, 2009, Am J Clin Nutr 89:1836-1845 disclose that using an isocaloric infant and follow on formula with a protein content of 1.77 and 2.1 g /100 kcal resulted in less weight gain than in the group of infants consuming an infant or follow on formula with a high protein concentration of 2.9 and 4.4 g/100 kcal. At 24 months, the weight-for-length z-score of infants in the lower protein formula group was lower than that of the high protein group and did not differ from that of the breast-fed reference group. In WO 2015/078505 a lower weight gain is observed in the 3 to 6 months period when a formula is administered comprising a lower protein content than in the control. WO 2015/091789 focusses on oligosaccharide mixtures comprising N-acetylated oligosaccharide, galacto-oligosaccharide and/or sialylated oligosaccharide promoting a rate of growth which approximates to the rate of growth of a breast-fed infants. WO 2012/1078039 relates to a fermented infant formulae for decreasing protein digestive effort by decreasing the amount of endogenously formed proteases, concomitant with an increased protein digestion. The improved digestion allows to reduce the protein levels. WO 2012/1078039 also relates to low protein fermented infant formulae. US 2014/0162223 provides methods for preventing and/or reducing early childhood obesity that may help instill early healthy eating habits and nutritious food preferences for infants and young children, promote an appropriate early growth trajectory, and a long term weight status that is consistent with public policy recommendations and associated with long term health. WO 2013/187764 discloses fermented infant formulae comprising non digestible oligosaccharides for improving intestinal tract health by decreasing protein digestive effort.

### SUMMARY OF THE INVENTION

An efficacy study on the growth and safety of a partly fermented infant formula during 3-4 months intervention was investigated in healthy, term infants compared to a standard formula and a breast-fed reference. In a randomized, controlled, multi-centre, double-blinded, prospective clinical trial, infants were enrolled before 28 days of age and assigned to receive one of two formulae until 17 weeks of age: 1) an infant formula comprising fermented formula or 2) a non-fermented infant formula. The composition of the formulas was similar in energy and macronutrient composition. As a reference, a group of infants was included being exclusively breast-fed until 17 weeks of age. Growth was evaluated by equivalence analysis of weight gain per day and BMI gain per day during the intervention period using equivalence margins of ± 0.5 SD, between formula groups as well as compared to the breast-fed reference group. Also length was monitored monthly.

At 17 weeks a statistically significant higher weight gain and BMI gain was observed for the control group, when comparing to the breast-fed reference group. There was no statistically significant difference in weight gain or BMI gain in infants fed with the fermented formula compared to the breast-fed reference group, nor compared to the infants fed with control formula.

At 17 weeks of age significant differences were observed in eating behaviours, in particular an increased satiety response, decreased food responsiveness and decreased general appetite was observed in the group receiving the fermented formula. This is indicative for improving the self-regulation of energy intake of the infant towards the self-regulation of energy intake observed in breast-fed infants.

Both the observed effect on the growth trajectories early in life being more similar to breast-fed reference group and on eating behaviour, are indicative for a decreased risk of becoming overweight or obese later in life in infants consuming a fermented formula.

Whereas in the art it is usually considered that a too high protein intake during infancy is a risk factor of becoming overweight and/or obese and hence reducing the level of protein in infant formula is considered beneficial, surprisingly the present inventors found that including a fermented ingredient in infant nutrition low in protein independently improved the effects on eating behaviour and growth trajectory and thereby improving the effect on prevention of overweight and/or obesity.

### DETAILED DESCRIPTION OF THE INVENTION

The present invention thus concerns a method for promoting improved eating behaviour and/or improved self-regulation of energy intake in an infant, said method comprising administering a nutritional composition selected from an infant formula and a follow on formula comprising 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria, and 0.05 to 1.5 wt% lactic acid based on dry weight.

In one embodiment, the present invention concerns a method for reducing the risk of overweight and/or obesity in an infant and/or preventing overweight and/or obesity in an infant, said method comprising administering a nutritional composition selected from an infant formula and a follow on formula comprising 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria, and 0.05 to 1.5 wt% lactic acid based on dry weight.

In some jurisdictions administering a nutritional composition to an infant is considered non-therapeutic. In those instances the invention can be worded as defined above by way of a method comprising administering a nutritional composition. For clarity, the method can also be defined as a non-therapeutic method for promoting improved eating behaviour and/or improved self-regulation of energy intake in an infant or as a non-therapeutic method for reducing the risk of overweight and/or obesity in an infant and/or preventing overweight and/or obesity in an infant. By definition, the words "non-therapeutic" exclude any therapeutic effect.

In some jurisdictions administering a nutritional composition to an infant is considered therapeutic per se. In this instances the invention can be worded as follows.

In one embodiment, the invention concerns the use of a fermented ingredient comprising lactic acid in the manufacture of a nutritional composition selected from an infant formula and a follow on formula for use in promoting improved eating behaviour and/or improved self-regulation of energy intake in an infant, wherein the nutritional composition comprises 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria and 0.05 to 1.5 wt% lactic acid based on dry weight.

The invention can also be worded as a nutritional composition selected from an infant formula and a follow on formula comprising 3 to 7 g lipid/100 kcal, 1.25 to 5 g protein/100 kcal and 6 to 18 g digestible carbohydrate/100 kcal and comprising an ingredient fermented by lactic acid producing bacteria and 0.05 to 1.5 wt% lactic acid based on dry weight, for use in promoting improved eating behaviour and/or improved self-regulation of energy intake in an infant.

In one embodiment, the invention concerns the use of a fermented ingredient comprising lactic acid in the manufacture of a nutritional composition selected from an infant formula and a follow on formula for use in reducing the risk of overweight and/or obesity in an infant and/or preventing overweight and/or obesity in an infant, wherein the nutritional composition comprises 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria and 0.05 to 1.5 wt% lactic acid based on dry weight.

The invention can also be worded as a nutritional composition selected from an infant formula and a follow on formula comprising 3 to 7 g lipid/100 kcal, 1.25 to 5 g protein/100 kcal and 6 to 18 g digestible carbohydrate/100 kcal and comprising an ingredient fermented by lactic acid producing bacteria and 0.05 to 1.5 wt% lactic acid based on dry weight, for use in reducing the risk of overweight and/or obesity in an infant and/or preventing overweight and/or obesity in an infant.

In the context of the present invention, the nutritional composition is not human milk.

### Fermented ingredient

The nutritional composition in the methods or uses according to the present invention, hereafter also referred to as the present nutritional composition, comprises a fermented ingredient. The presence of the fermented ingredient in the nutritional composition improves in infants the eating behaviour and/or the self-regulation of energy intake. Therefore, the presence of the fermented ingredient in the nutritional composition reduces the risk of becoming overweight or obese.

The fermented ingredient is a composition that is fermented by lactic acid producing bacteria. The fermentation preferably takes place during the production process of the nutritional composition. Preferably, the nutritional composition does not contain significant amounts of viable bacteria in the final product due to heat inactivation after fermentation or inactivation by other means. Preferably the fermented ingredient is a milk-derived product, which is a milk substrate that is fermented by lactic acid producing bacteria, and said milk substrate comprising at least one selected from the group consisting of milk, whey, whey protein, whey protein hydrolysate, casein, casein hydrolysate, and lactose or mixtures thereof. Suitably, nutritional compositions comprising fermented ingredient and non-digestible oligosaccharide and their way of producing them are described in WO 2009/151330, WO 2009/151331 and WO 2013/187764.

The fermented ingredient preferably comprises bacterial cell fragments like glycoproteins, glycolipids, peptidoglycan, lipoteichoic acid (LTA), lipoproteins, nucleotides, and/or capsular polysaccharides. Furthermore, upon fermentation and/or other interactions of lactic acid producing bacteria with the milk substrate, additional bio-active compounds are formed, such as bioactive peptides and/or oligosaccharides and organic acids. Furthermore, fermented formula improve the efficacy of protein digestion. Therefore the fermented ingredient, in particular fermented milk-derived product, is believed to have an improved effect compared to non-fermented ingredient, in particular non fermented milk-derived product.

Preferably the nutritional composition comprises 5 to 97.5 wt% of the fermented ingredient based on dry weight, more preferably 10 to 95 wt% , more preferably 20 to 90 wt%, even more preferably 25 to 60 wt%. As a way to specify the extent of fermentation, the level of the sum of lactic acid and lactate in the nutritional composition can be taken, as this is the metabolic end product produced by the lactic acid producing bacteria upon fermentation. The present nutritional composition comprises 0.05 to 1.5 wt% of the sum of lactic acid and lactate based on dry weight of the composition, more preferably 0.05 to 1.0 wt%, even more preferably 0.1 to 0.75 wt%, even more preferably 0.1 to 0.6 wt%, even more preferably 0.1 to 0.5 wt%. Preferably at least 50 wt%, even more preferably at least 90 wt%, of the sum of lactic acid and lactate is in the form of the L(+)-isomer. Thus in one embodiment the sum of L(+)-lactic acid and L(+)-lactate is more than 50 wt%, more preferably more than 90 wt%, based on the sum of total lactic acid and lactate. Herein L(+)-lactate and L(+)-lactic acid is also referred to as L-lactate and L-lactic acid.

### Lactic acid producing bacteria used for producing the fermented ingredient

Lactic acid producing bacteria used for preparing the fermented ingredient, in particular for fermentation of the milk substrate are preferably provided as a mono- or mixed culture. Lactic acid producing bacteria consists of the genera *Bifidobacterium*, *Lactobacillus*, *Carnobacterium*, *Enterococcus, Lactococcus, Leuconostoc, Oenococcus, Pediococcus, Streptococcus, Tetragenococcus*, *Vagococcus and Weissella.* Preferably the lactic acid producing bacteria used for fermentation comprises bacteria of the genus *Bifidobacterium* and/or *Streptococcus.*

Preferably the *Streptococcus* is a strain of *S. thermophilus.* Preferably the *Streptococcus* develops beta-galactosidase activity in the course of fermentation of the substrate. Selection of a suitable strain *of S. thermophilus* is described in example 2 of EP 778885 and in example 1 of FR 2723960. In a further preferred embodiment according to the present invention, the nutritional composition comprises 10³-10⁶ cfu living bacteria *of S. thermophilus,* per g dry weight of the nutritional composition, preferably the nutritional composition comprises 10³-10⁵ living bacteria *of S. thermophilus* per g dry weight.

Preferred strains *of S. thermophilus* to prepare the fermented ingredient for the purpose of the present invention have been deposited by Compagnie Gervais Danone at the Collection Nationale de Cultures de Microorganismes (CNCM) run by the Institut Pasteur, 25 rue du Docteur Roux, Paris, France on 23 August 1995 under the accession number 1-1620 and on 25 August 1994 under the accession number 1-1470.

Bifidobacteria are Gram-positive, anaerobic, rod-shaped bacteria. Preferably the lactic acid producing bacteria used for fermentation comprises or is at least one *Bifidobacterium* selected from the group consisting of *B*. *breve*, *B. infantis*, *B*. *bifidum*, *B. catenulatum*, *B. adolescentis*, *B*. *thermophilum*, *B. gallicum*, *B. animalis or lactis*, *B*. *angulatum*, *B*. *pseudocatenulatum*, *B*. *thermacidophilum* and B. *longum* more preferably *B. breve*, *B. infantis*, *B. bifidum*, *B. catenulatum*, *B. longum*, more preferably *B. longum* and *B. breve,* even more preferably *B. breve*, more preferably *B. breve* selected from the group consisting of *B. breve* Bb-03 (Rhodia/Danisco), *B. breve* M-16V (Morinaga), *B. breve* R0070 (Institute Rosell, Lallemand), *B. breve* BR03 (Probiotical), *B. breve* BR92 (Cell Biotech) DSM 20091, LMG 11613 and *B. breve* 1-2219 deposited at the CNCM, Paris France. Most preferably, the *B. breve* is *B. breve* M-16V (Morinaga) or *B. breve* 1-2219, even more preferably *B. breve* I-2219.

Most preferably the nutritional composition comprises fermented ingredient that is fermented by lactic acid bacteria comprising both *B. breve* and *S. thermophilus.* In one embodiment, the fermentation by lactic acid bacteria, is fermentation by *Streptococcus thermophilus* and *Bifidobacterium breve.* In one embodiment, the nutritional composition comprises fermented ingredient wherein the lactic acid bacteria are inactivated after fermentation.

Preferably the fermented ingredient is not fermented by *Lactobacillus bulgaricus. L. bulgaricus* fermented products are considered not suitable for infants, since in young infants the specific dehydrogenase that converts D-lactate to pyruvate is far less active than the dehydrogenase which converts L-lactate.

Preferably the present nutritional composition comprises inactivated lactic acid producing bacteria and/or bacterial fragments derived from lactic acid producing bacteria obtained from more than 1x10⁴ cfu lactic acid producing bacteria per g based on dry weight of the final composition, more preferably more than 1x10⁵ cfu, even more preferably more than 1x10⁶ cfu. Preferably the inactivated bacteria or bacterial fragments are obtained from less than 1x10¹⁴ cfu lactic acid producing bacteria per g based on dry weight of the final composition, more preferably less than 1x10¹³ cfu, even more preferably less than 1x10¹² cfu.

### Process of fermentation

The milk substrate to be fermented is suitably present in an aqueous medium. The milk substrate to be fermented is preferably selected from the group consisting of milk, whey, whey protein, whey protein hydrolysate, casein, casein hydrolysate, and lactose, and mixtures thereof. Milk can be whole milk, semi-skimmed milk and/or skimmed milk. Preferably the milk substrate to be fermented comprises skimmed milk. Whey can be sweet whey, and/or acid whey. Preferably the whey is present in a concentration of 3 to 80 g dry weight per 1 aqueous medium containing milk substrate, more preferably 40 to 60 g per 1. Preferably whey protein hydrolysate is present in 2 to 80 g dry weight per 1 aqueous medium containing milk substrate, more preferably 5 to15 g/l. Preferably lactose is present in 5 to 50 g dry weight per 1 aqueous substrate, more preferably 1 to 30 g/l. Preferably the aqueous medium containing milk substrate comprises buffer salts in order to keep the pH within a desired range. Preferably sodium or potassium dihydrogen phosphate is used as buffer salt, preferably in 0.5 to 5 g/l, more preferably 1.5 to 3 g per 1. Preferably the aqueous medium containing milk substrate comprises cysteine in amount of 0.1 to 0.5 g per 1 aqueous substrate, more preferably 0.2 to 0.4 g/l. The presence of cysteine results in low redox potential of the substrate which is advantageous for activity of lactic acid producing bacteria, particularly bifidobacteria. Preferably the aqueous medium containing milk substrate comprises yeast extract in an amount of 0.5 to 5 g/l aqueous medium containing milk substrate, more preferably 1.5 to 3 g/l. Yeast extract is a rich source of enzyme co-factors and growth factors for lactic acid producing bacteria. The presence of yeast extract will enhance the fermentation by lactic acid producing bacteria.

Suitably the milk substrate, in particular the aqueous medium containing milk substrate, is pasteurised before the fermentation step, in order to eliminate the presence of unwanted living bacteria. Suitably the product is pasteurised after fermentation, in order to inactivate enzymes. Suitably the enzyme inactivation takes place at 75 °C for 3 min. Suitably the aqueous medium containing milk substrate is homogenised before and/or the milk-derived product is homogenised after the fermentation. Homogenisation results in a more stable substrate and/or fermented product, especially in the presence of fat.

The inoculation density is preferably between 1x10² to 5x10¹⁰, preferably between 1x10⁴ to 5x10⁹ cfu lactic acid producing bacteria/ml aqueous medium containing milk substrate, more preferably between 1x10⁷ to 1x10⁹ cfu lactic acid producing bacteria/ml aqueous medium containing milk substrate. The final bacteria density after fermentation is preferably between 1x10³ to 1x10¹⁰, more preferably between 1x10⁴ to 1x10⁹ cfu/ml aqueous medium containing milk substrate.

The fermentation is preferably performed at a temperature of approximately 20 °C to 50 °C, more preferably 30 °C to 45 °C, even more preferably approximately 37 °C to 42 °C. The optimum temperature for growth and/or activity for lactic acid producing bacteria, more particularly lactobacilli and/or bifidobacteria is between 37 °C and 42 °C.

The incubation is preferably performed at a pH of 4 to 8, more preferably 6 to 7.5. This pH does not induce protein precipitation and/or an adverse taste, while at the same time lactic acid producing bacteria such as lactobacilli and/or bifidobacteria are able to ferment the milk substrate.

The incubation time preferably ranges from 10 minutes to 48 h, preferably from 2 h to 24 h, more preferably from 4 h to 12 h. A sufficient long time enables the fermentation and the concomitant production of cell fragments, bioactive compounds, and organic acids to take place at a high extent, whereas the incubation time need not be unnecessarily long for economic reasons.

Procedures to prepare fermented ingredients suitable for the purpose of the present invention are known per se. EP 778885, which is incorporated herein by reference, discloses in particular in example 7 a suitable process for preparing a fermented ingredient. FR 2723960, which is incorporated herein by reference, discloses in particular in example 6 a suitable process for preparing a fermented ingredient. Briefly, a milk substrate, preferably pasteurised, containing lactose and optionally further macronutrients such as fats, preferably vegetable fats, casein, whey protein, vitamins and/or minerals etc. is concentrated, e.g. to between 15 to 50% dry matter and then inoculated with *S. thermophilus,* for example with 5% of a culture containing 10⁶ to 10¹⁰ bacteria per ml. Preferably this milk substrate comprises milk protein peptides. Temperature and duration of fermentation are as mentioned above. Suitably after fermentation the fermented ingredient may be pasteurised or sterilized and for example spray dried or lyophilised to provide a form suitable to be formulated in the end product.

A preferred method for preparing the fermented ingredient of the present invention is disclosed in WO 01/01785, more particular in examples 1 and 2. A preferred method for preparing the fermented product of the present invention is described in WO 2004/093899, more particularly in example 1.

### Methods of inactivation and/or physically removal of living cells

Living cells of lactic acid producing bacteria in the fermented ingredient are after fermentation preferably eliminated, for example by inactivation and/or physical removal. The cells are preferably inactivated. The cells are preferably treated to become non-replicating. Preferably the lactic acid producing bacteria are heat killed after fermentation of the milk substrate. Preferable ways of heat killing are (flash) pasteurization, sterilization, ultra high temperature treatment, high temperature/short time heat treatment, and/or spray drying at temperatures bacteria do not survive or are no longer able to replicate. Cell fragments are preferably obtained by heat treatment. With this heat treatment preferably at least 90 % of living microorganisms are inactivated, more preferably at least 95 %, even more preferably at least 99 %. Preferably the fermented nutritional composition comprises less than 1x10⁵ colony forming units (cfu) living lactic acid bacteria per g dry weight, more preferably less than 1x10⁴, even more preferably less than 1x10³ cfu living lactic acid bacteria per g dry weight. The heat treatment preferably is performed at a temperature ranging from 70 to180 °C, preferably from 80 to 150 °C, preferably for about 3 minutes to 2 hours, preferably in the range of 80 to 140 °C for 5 minutes to 40 minutes. Inactivation of the lactic acid bacteria advantageously results in less post acidification and a safer product. This is especially advantageous when the nutritional composition is to be administered to infants or young children. Suitably after fermentation the fermented ingredient may be pasteurised or sterilized and for example spray dried or lyophilised to provide a form suitable to be formulated in the end product.

### Non-digestible oligosaccharide

The present nutritional composition comprises non-digestible oligosaccharide and preferably comprises at least two different non-digestible oligosaccharides, in particular two different sources of non-digestible oligosaccharide. The presence of non-digestible oligosaccharide aides in promoting an improved eating behaviour or self-regulation of energy take. The presence of non-digestible oligosaccharides beneficially affects the gut microbiota, and makes it more similar to the gut microbiota of breast-fed infants. Such microbiota is correlated to a reduced risk of obesity. Combining fermented ingredient and non-digestible oligosaccharides will further improve the claimed benefits of the invention.

The term "oligosaccharide" as used herein refers to saccharides with a degree of polymerization (DP) of 2 to 250, preferably a DP 2 to 100, more preferably 2 to 60, even more preferably 2 to 10. If oligosaccharide with a DP of 2 to 100 is included in the present nutritional composition, this results in compositions that may contain oligosaccharides with a DP of 2 to 5, a DP of 50 to 70 and a DP of 7 to 60. The term "non-digestible oligosaccharide" as used in the present invention refers to oligosaccharides which are not digested in the intestine by the action of acids or digestive enzymes present in the human upper digestive tract, e.g. small intestine and stomach, but which are preferably fermented by the human intestinal microbiota. For example, sucrose, lactose, maltose and maltodextrins are considered digestible.

Preferably the present non-digestible oligosaccharide is soluble. The term "soluble" as used herein, when having reference to a polysaccharide, fibre or oligosaccharide, means that the substance is at least soluble according to the method described by L. Prosky et al., J. Assoc. Off. Anal. Chem. 71, 1017-1023 (1988).

The non-digestible oligosaccharide included in the present nutritional compositions in the methods or uses according to the present invention preferably include a mixture of non-digestible oligosaccharides. The non-digestible oligosaccharide is preferably selected from the group consisting of fructo-oligosaccharide, such as inulin, non-digestible dextrins, galacto-oligosaccharide, such as transgalacto-oligosaccharide, xylo-oligosaccharide, arabino-oligosaccharide, arabinogalacto-oligosaccharide, gluco-oligosaccharide, gentio-oligosaccharide, glucomanno-oligosaccharide, galactomanno-oligosaccharide, mannan-oligosaccharide, isomalto-oligosaccharide, nigero-oligosaccharide, glucomanno-oligosaccharide, chito-oligosaccharide, soy oligosaccharide, uronic acid oligosaccharide, sialyloligosaccharide, such as 3-sialyllactose (3-SL), 6-sialyllactose (6-SL), lactosialyltetrasaccharide a,b,c (LST), disialyllactoNtetraose (DSLNT), sialyl-lactoNhexaose (S-LNH), DS-LNH, and fuco-oligosaccharide, such as (un)sulphated fucoidan oligosaccharide, 2'-fucosyllactose (2'-FL), 3-fucosyllactose (3-FL), difucosyllactose, lacto-N-fucopenatose, (LNFP) I, II, III, V, Lacto-N-neofucopenaose (LNnFP), Lacto-N-difucosyl-hexaose (LNDH), and mixtures thereof, even more preferably selected from the group consisting of fructo-oligosaccharide, such as inulin, galacto-oligosaccharide, such as transgalacto-oligosaccharide, and fuco-oligosaccharide and mixtures thereof, even more preferably transgalacto-oligosaccharide, and/or inulin, most preferably transgalacto-oligosaccharide. In one embodiment in the composition or methods according to the present invention, the non-digestible oligosaccharide is selected from the group consisting of transgalacto-oligosaccharide, fructo-oligosaccharide and mixtures of thereof.

The non-digestible oligosaccharide is preferably selected from the group consisting of β-galacto-oligosaccharide, α-galacto-oligosaccharide, and galactan. According to a more preferred embodiment non-digestible oligosaccharide is β-galacto-oligosaccharide. Preferably the non-digestible oligosaccharide comprises galacto-oligosaccharide with β(1,4), β(1,3) and/or β(1,6) glycosidic bonds and a terminal glucose. Transgalacto-oligosaccharide is for example available under the trade name Vivinal®GOS (Domo FrieslandCampina Ingredients), Bi2muno (Clasado), Cup-oligo (Nissin Sugar) and Oligomate55 (Yakult).

The non-digestible oligosaccharide preferably comprises fructo-oligosaccharide. A fructo-oligosaccharide may in other context have names like fructopolysaccharide, oligofructose, polyfructose, polyfructan, inulin, levan and fructan and may refer to oligosaccharides comprising β-linked fructose units, which are preferably linked by β(2,1) and/or β(2,6) glycosidic linkages, and a preferable DP between 2 and 200. Preferably, the fructo-oligosaccharide contains a terminal β(2,1) glycosidic linked glucose. Preferably, the fructo-oligosaccharide contains at least 7 β-linked fructose units. In a further preferred embodiment inulin is used. Inulin is a type of fructo-oligosaccharide wherein at least 75% of the glycosidic linkages are β(2,1) linkages. Typically, inulin has an average chain length between 8 and 60 monosaccharide units. A suitable fructo-oligosaccharide for use in the compositions of the present invention is commercially available under the trade name Raftiline®HP (Orafti). Other suitable sources are Raftilose (Orafti), Fibrulose and Fibruline (Cosucra) and Frutafit and Frutalose (Sensus).

Preferably the present nutritional composition comprises a mixture of galacto-oligosaccharide and fructo-oligosaccharide. Preferably the mixture of galacto-oligosaccharide and fructo-oligosaccharide is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when galacto-oligosaccharide has a low average DP and fructo-oligosaccharide has a relatively high DP. Most preferred is a mixture of galacto-oligosaccharide with an average DP below 10, preferably below 6 and a fructo-oligosaccharide with an average DP above 7, preferably above 11, even more preferably above 20. Such a mixture synergistically improves the gut microbiota.

Preferably the present nutritional composition comprises a mixture of short chain fructo-oligosaccharide and long chain fructo-oligosaccharide. Preferably the mixture of short chain fructo-oligosaccharide and long chain fructo-oligosaccharide is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharide with an average DP below 10, preferably below 6 and a fructo-oligosaccharide with an average DP above 7, preferably above 11, even more preferably above 20.

The present nutritional composition comprises 2.5 to 20 wt% total non-digestible oligosaccharide, more preferably 2.5 to 15 wt%, even more preferably 3.0 to 10 wt%, most preferably 5.0 to 7.5 wt%, based on dry weight of the nutritional composition. Based on 100 ml the present nutritional composition preferably comprises 0.35 to 2.5 wt% total non-digestible oligosaccharide, more preferably 0.35 to 2.0 wt%, even more preferably 0.4 to 1.5 wt%, based on 100 ml of the nutritional composition. A lower amount of non-digestible oligosaccharide will be less effective in promoting improved eating behaviour or improving gut microbiota, whereas a too high amount will result in side-effects of bloating and abdominal discomfort.

### Infant formula and follow on formula

The nutritional composition to be administered in the method or use according to the present invention is selected from an infant formula and a follow on formula. This means that the present nutrition composition is not human milk. Alternatively the term "formula" means that it concerns a composition that is artificially made or in other words that it is synthetic. Hence in one embodiment the nutritional composition is selected from an artificial infant formula and an artificial follow on formula or a synthetic infant formula and a synthetic follow on formula. In the present context, infant formula refers to nutritional compositions, artificially made, intended for infants of 0 to about 4 to 6 months of age and are intended as a substitute for human milk. Typically infant formulae are suitable to be used as sole source of nutrition. Such formulae are also known as starter formula. Formula for infants starting with at 4 to 6 months of life to 12 months of life are intended to be supplementary feedings to infants that start weaning on other foods. Such formulae are also known as follow on formulae. Infant and follow on formulae are subject to strict regulations, for example for the EU Commission Directive 2006/141/EC.

The nutritional composition comprises 3 to 7 g lipid/100 kcal, preferably 4 to 6 g lipid/100 kcal, more preferably 4.5 to 5.5 g lipid/100 kcal, 1.25 to 5 g protein/100 kcal, preferably 1.25 to 4 g protein/100 kcal, more preferably 1.25 to 3 g protein/100 kcal, more preferably 1.25 to 2.5 g protein/100 kcal, more preferably 1.25 to 2.25 g/100 kcal, even more preferably 1.25 to 2.1 g protein/100 kcal, even more preferably 1.6 to 2.0 g protein/100 kcal even more preferably 1.7 to 2.0 g protein/100 kcal, even more preferably 1.75 to 1.95 g protein/100 kcal and 6 to 18 g digestible carbohydrate/100 kcal, preferably 8 to 16 g digestible carbohydrate/100 kcal, more preferably 10 to 15 g digestible carbohydrate/100 kcal.

### Lipid

Herein LA refers to linoleic acid and/or acyl chain (18:2 n6); ALA refers to α-linolenic acid and/or acyl chain (18:3 n3); PUFA refers to polyunsaturated fatty acids and/or acyl chains; MUFA refers to monounsaturated fatty acids and/or acyl chains; LC-PUFA refers to long chain polyunsaturated fatty acids and/or acyl chains comprising at least 20 carbon atoms in the fatty acyl chain and with 2 or more unsaturated bonds; DHA refers to docosahexaenoic acid and/or acyl chain (22:6, n3); EPA refers to eicosapentaenoic acid and/or acyl chain (20:5 n3); ARA refers to arachidonic acid and/or acyl chain (20:4 n6); DPA refers to docosapentaenoic acid and/or acyl chain (22:5 n3). PA relates to palmitic acid and/or acyl chains (C16:0). Medium chain fatty acids (MCFAs) refer to fatty acids and/or acyl chains with a chain length of 6, 8 or 10 carbon atoms.

The lipid in the nutritional composition to be administered in the method or use according to the present invention preferably comprises vegetable lipids. The lipid that is present in the nutritional composition in the method or use according to the invention preferably comprises PUFAs, more preferably LC-PUFAs, as LC-PUFAs further improve the development of the infant. The nutritional composition preferably comprises 5 to 35 wt.% PUFA, more preferably 10 to 30 wt.% PUFA, most preferably 15 to20 wt.% PUFA, based on total lipid. In one embodiment the lipid in the nutritional composition for the method or use according to the invention comprises at least 10 wt.% polyunsaturated fatty acid based on total fatty acids. It is also preferred that the nutritional composition comprises MUFAs, preferably 10 to 80 wt.% MUFA, more preferably 20 to 70 wt.% MUFA, most preferably 35 to 55 wt.% MUFA, based on total lipid.

LA preferably is present in a sufficient amount in order to promote a healthy growth and development, yet in an amount as low as possible to prevent occurrence of unbalance in growth or body development. The nutritional composition therefore preferably comprises less than 20 wt.% LA based on total lipid, preferably 5 to 16 wt.%, more preferably 10 to 14.5 wt.%. Preferably, the nutritional composition comprises at least 5 wt.% LA based on total lipid. Per 100 kcal, the nutritional composition preferably comprises 350 - 1400 mgLA. Preferably, ALA is present in a sufficient amount to promote a healthy growth and development of the infant. The nutritional composition therefore preferably comprises at least 1.0 wt.% ALA based on total lipid. Preferably the nutritional composition comprises at least 1.5 wt.% ALA based on total lipid, more preferably at least 2.0 wt.%. Preferably the nutritional composition comprises less than 12.5 wt.% ALA, more preferably less than 10.0 wt.%, most preferably less than 5.0 wt.%. Preferably the nutritional composition comprises a weight ratio of LA/ALA from 2 to 20, more preferably from 3 to 16, more preferably from 4 to 14, more preferably from 5 to 12.

According to the present invention, the nutritional composition preferably comprises LC-PUFA, more preferably n-3 LC-PUFA, since n-3 LC-PUFA promote advantageous growth. More preferably, the nutritional composition comprises EPA, DPA and/or DHA, even more preferably DHA. Since a low concentration of DHA, DPA and/or EPA is already effective and normal growth and development are important, the content of n-3 LC-PUFA in the nutritional composition preferably does not exceed 15 wt.% of the total fatty acid content, preferably does not exceed 10 wt.%, even more preferably does not exceed 5 wt.%. Preferably the nutritional composition comprises at least 0.15 wt.%, preferably at least 0.35 wt.%, more preferably at least 0.75 wt.%, n-3 LC-PUFA based on fatty acid content. The content of DHA in the nutritional composition preferably does not exceed 10 wt.% of the total fatty acid content, preferably does not exceed 5 wt.%, Preferably the nutritional composition comprises at least 0.15 wt.%, preferably at least 0.35 wt.%, wt.% of DHA based on total fatty acids.

As the group of n-6 fatty acids, especially arachidonic acid (ARA) and LA as its precursor, counteracts the group of n-3 fatty acids, especially DHA and EPA and ALA as their precursor, the nutritional composition comprises relatively low amounts of ARA. The n-6 LC-PUFA, more preferably ARA, content preferably does not exceed 5 wt.%, more preferably does not exceed 2.0 wt.%, more preferably does not exceed 0.75 wt.%, even more preferably does not exceed 0.5 wt.%, based on total fatty acids. ARA may also be absent.

### Protein

The nutritional composition comprises proteins, preferably in the amounts of 1.25 to 5 g protein/100 kcal, preferably 1.25 to 4 g protein/100 kcal, more preferably 1.25 to 3 g protein/100 kcal, more preferably 1.25 to 2.5 g protein/100 kcal, more preferably 1.25 to 2.25 g/100 kcal, even more preferably 1.25 to 2.1 g protein/100 kcal, even more preferably 1.6 to 2.1 g protein/100 kcal, even more preferably 1.6 to 2.0 g protein/100 kcal, even more preferably 1.7 to 2.0 g protein/100 kcal, even more preferably 1.75 to 1.95 g protein/100 kcal. An optimal amount of protein will beneficially affect eating behaviour. The combination of a fermented ingredient and the optimal amount of protein has a further improved effect on eating behaviour and/or self-regulation of energy intake. The source of the protein should be selected in such a way that the minimum requirements for essential amino acid content are met and satisfactory growth is ensured. Hence protein sources based on cows' milk proteins such as whey, casein and mixtures thereof and proteins based on soy, potato or pea are preferred. In case whey proteins are used, the protein source is preferably based on acid whey or sweet whey, whey protein isolate or mixtures thereof and may include α-lactalbumin and β-lactoglobulin. More preferably, the protein source is based on acid whey or sweet whey from which caseino-glyco-macropeptide (CGMP) has been removed. Preferably the composition comprises at least 3 wt.% casein based on dry weight. Preferably the casein is intact and/or non-hydrolyzed. For the present invention protein includes peptides and free amino acids.

### Digestible carbohydrates

The nutritional composition comprises digestible carbohydrate, preferably in the amounts specified above. Preferred digestible carbohydrate sources are lactose, glucose, sucrose, fructose, galactose, maltose, starch and maltodextrin. Lactose is the main digestible carbohydrate present in human milk. Lactose advantageously has a low glycemic index. The nutritional composition preferably comprises lactose. The nutritional composition preferably comprises digestible carbohydrate, wherein at least 35 wt.%, more preferably at least 50 wt.%, more preferably at least 75 wt.%, even more preferably at least 90 wt.%, most preferably at least 95 wt.% of the digestible carbohydrate is lactose. Based on dry weight the present composition preferably comprises at least 25 wt.% lactose, preferably at least 40 wt.%.

### Application

In the method or use according to the present invention, a nutritional composition is administered to an infant or is used in an infant. In the context of the present invention an infant has an age up to 12 months. Preferably the nutritional composition is administered to or is used in a term infant. A term infant means an infant born at a gestational age of ≥37 to ≤42 weeks. Preferably the nutritional composition is administered to or is used in a healthy infant. Preferably the nutritional composition is used at least during the first 2 months of life, preferably at least during the first 3 months of life of the infant, more preferably at least during the first 4 months of life of the infant. Preferably the nutritional composition is administered to an infant with an age below 6 months, more preferably below 4 months of age.

In one embodiment, the present nutritional composition is of particular benefit for infants that are exposed to an obesogenic environment or in other words that are exposed to a Western type diet later on.

An obesogenic environment, or obesogenic diet, or Western type diet is high in calories, high in fat and high in sugar. The fat is high in saturated fat, it has a high n6/n3 fatty acid ratio and is high in cholestrol. The diet is generally characterized by a high intake in processed meat, red meat, butter, high fat dairy products, sugar and refined grains. WHO/FAO has guidelines for the recommended diet and the Western type diet is deviating from that guidelines, FAO (Food and Agriculture Organization of the United Nations) Food and Nutrition Paper 91: Fats and fatty acids in human nutrition - Report of an expert consultation, held 10-14 November 2008 in Geneva, available in print November 2010, ISBN 978-92-5-106733-8. Western type diet is sometimes also referred to as Standard American Diet. For the purpose of the present invention, Western food or in other words a Western type diet is preferably characterised by 1) that over 30% of the total calories is provided by fat, 2) that it comprises at least 10 wt.% saturated fat based on total amount of fat, 3) that it comprises at least 0.5 wt% cholesterol based on total fat, 4) that the n6/n3 ratio of the fatty acids in the dietary fat is above 4, and in an improved definition the n6/n3 ratio of the fatty acids in the dietary fat is above 10.

Thus according to one embodiment of the present invention the nutritional composition is to be used in an infant that is exposed to an obesogenic environment and/or a Western type diet later in life, preferably when the infant has an has an age above 36 months. The present invention is therefore in particular intended for infants at risk of becoming obese later in life. The present invention is therefore in particular intended for infants raised in an obesogenic environment. An obesogenic environment is an environment wherein humans are surrounded by cheap, fast, nutritionally inferior food and a built environment that discourages physical activity.

The present invention also concerns improving the self-regulation of energy intake of the infant towards the self-regulation of energy intake observed in breast-fed infants, preferably when compared to self-regulation of energy intake in infants fed infant formula of follow on formula that does not comprise fermented ingredient as defined herein, or in other words that does not comprise lactic acid in the range as defined herein. In a preferred embodiment the improved eating behaviour is selected from the group consisting of an increased slowness of eating, an increased satiety responsiveness, a decreased food responsiveness, an optimal enjoyment of food, and an optimal appetite, more preferably an increased satiety responsiveness, a decreased food responsiveness and a decreased general appetite, preferably when compared to the rate of eating, satiety responsiveness, food responsiveness, level of enjoyment of food, and/or level of appetite in infants fed infant formula of follow on formula that does not comprise fermented ingredient as defined herein, or in other words that does not comprise lactic acid in the range as defined herein.

In one embodiment according to the present invention a reduction in risk of becoming overweight and/or obese, and/or preventing overweight and/or obesity in an infant is promoted. More preferably, a reduction in risk of becoming overweight and/or obese later in life, and/or preventing overweight and/or obesity in an infant later in life is promoted. More preferably a reduction in risk of childhood obesity, and/or preventing childhood obesity is promoted in an infant.

### LIST OF EMBODIMENTS

1. A non-therapeutic method for promoting improved eating behaviour and/or improved self-regulation of energy intake in an infant, said method comprising administering a nutritional composition selected from an infant formula and a follow on formula comprising 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria, and 0.05 to 1.5 wt% lactic acid based on dry weight.
2. The non-therapeutic method according to embodiment 1 wherein the improved eating behaviour is selected from the group consisting of an increased slowness of eating, an increased satiety responsiveness, a decreased food responsiveness, an optimal enjoyment of food, and an optimal appetite, more preferably an increased satiety responsiveness, a decreased food responsiveness and a decreased general appetite.
3. Use of a fermented ingredient comprising lactic acid in the manufacture of a nutritional composition selected from an infant formula and a follow on formula for use in reducing the risk of overweight and/or obesity in an infant and/or preventing overweight and/or obesity in an infant, wherein the nutritional composition comprises 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria and 0.05 to 1.5 wt% lactic acid based on dry weight.
4. The non-therapeutic method according to embodiment 1 or 2 or use according to embodiment 3, wherein the nutritional composition comprises 1.6 to 2.1 g protein/100 kcal.
5. The non-therapeutic method or use according to any one of the preceding embodiments, wherein the nutritional composition comprises 0.25 wt% to 20 wt% of non-digestible oligosaccharides based on dry weight of the nutritional composition.
6. The non-therapeutic method or use according to embodiment 5, wherein the non-digestible oligosaccharides are selected from the group consisting of fructo-oligosaccharides and galacto-oligosaccharides.
7. The non-therapeutic method or use according to any one of the preceding embodiments wherein the lactic acid producing bacteria are *Bifidobacterium breve* and/or *Streptococcus thermophilus.*
8. The non-therapeutic method or use according to any one of the preceding embodiments, wherein the nutritional composition comprises inactivated lactic acid producing bacteria.
9. The non-therapeutic method or use according to any one of the preceding embodiments, wherein the nutritional composition is an infant formula.
10. The non-therapeutic method or use according to any one of the preceding embodiments, wherein the nutritional composition comprises 1.7 to 2.0 g protein/100 kcal.
11. The non-therapeutic method or use according to any one of the preceding embodiments, wherein the nutritional composition comprises 1.75 to 1.95 g protein/100 kcal.
12. The non-therapeutic method or use according to any one of the preceding embodiments, wherein the nutritional composition comprises lactic acid and lactate in an amount of 0.1 to 0.75 wt% based on dry weight of the composition.
13. The non-therapeutic method or use according to any one of the preceding embodiments, wherein the infant is exposed to an obesogenic environment and/or a Western type diet later in life.
14. A method for reducing the risk of overweight and/or obesity in an infant and/or preventing overweight and/or obesity in an infant, said method comprising administering a nutritional composition selected from an infant formula and a follow on formula comprising 3 to 7 g lipid/100 kcal, 1.25 to 5 g protein/100 kcal and 6 to 18 g digestible carbohydrate/100 kcal and wherein the nutritional composition comprises an ingredient fermented by lactic acid producing bacteria, and 0.05 to 1.5 wt% lactic acid based on dry weight.
15. A nutritional composition selected from an infant formula and a follow on formula comprising 3 to 7 g lipid/100 kcal, 1.25 to 5 g protein/100 kcal and 6 to 18 g digestible carbohydrate/100 kcal and comprising an ingredient fermented by lactic acid producing bacteria and 0.05 to 1.5 wt% lactic acid based on dry weight for use in reducing the risk of overweight and/or obesity in an infant and/or preventing overweight and/or obesity in an infant.

### EXAMPLES

### Example 1: Early in life growth trajectories and body composition development in infants consuming partly fermented infant formula or control formula compared with a breast-fed reference group

Growth and safety of an experimental formula (formula 1) versus control formula (formula 2) was investigated in an explorative clinical study, using a 3-4 months intervention in healthy, term infants. In a randomized, controlled, multi-centre, double-blinded, prospective clinical trial, infants were enrolled before 28 days of age and assigned to receive one of two formulae until 17 weeks of age.

Experimental infant formula 1 is an infant formula containing 0.8g/100ml non-digestible oligosaccharides of scGOS (source Vivinal® GOS) and lcFOS (source RaftilinHP®) in a 9:1 wt ratio. Of this infant formula 30% based on dry weight was derived from Lactofidus™, a commercially available infant formula marketed under brand name Gallia. Lactofidus™ is a fermented milk derived composition and is produced by fermenting with *S. thermophilus* and comprises *B. breve.* A mild heat treatment is employed. The infant formula 1 comprised about 0.33 wt.% lactic acid + lactate based on dry weight, of which at least 95% is L-lactic acid + L-lactate.
Control infant formula 2 is a non-fermented infant formula without scGOS/lcFOS.
The composition of the two formulae was similar in energy and macronutrient composition (per 100ml: 66 kcal, 1.2 g protein (bovine whey protein/casein in 1/1 weight ratio), 7.7 g digestible carbohydrate (of which 7.6 g lactose), 3.4 g fat (mainly vegetable fat), The composition further comprised vitamins, minerals, trace elements and other micronutrients according to international directive 2006/141/EC for infant formula.

As a reference, a group of infants was included being exclusively breast-fed until 17 weeks of age. The Intention-To-Treat (ITT) population consisted of all subjects randomised to infant formula (n=199, in addition 100 subjects were include in the breast-fed reference group. The ITT population consisted of 94 subjects in the experimental group, 105 subjects in the control group. The Per Protocol (PP) population consisted of all subjects from the ITT group without predetermined protocol deviations. For the analysis of growth data, the PP analysis was considered to be the leading (principal) analysis, this population consisted of 145 randomized subjects (71 in the experimental group and 74 in the control group) and 86 breast-fed subjects. A total of 75 subject terminated the study prematurely, n=22 for the experimental group, n=25 for the control group (no significant difference between the groups) and n=28 in the breast-fed reference group. The reasons for early termination were subjects with an AE (n=5 for experimental, n=9 for control, n=0 for breast-fed), withdrew consent (n=2 for experimental, n=3 for control, n=17 for breast-fed), lost for follow-up (n=4 for experimental, n=1 for control, n=7 for breast-fed) and other (n=11 for experimental, n=11 for control, n=4 for breast-fed). There were no differences between experimental and control group with respect to baseline characteristics, except that there were more twins in the control group. There were no differences between experimental and control group with respect to parental characteristics.

Growth was evaluated by equivalence analysis of weight gain per day during the intervention period using equivalence margins of ± 0.5 SD, between formula groups (per protocol) as well as compared to the breast-fed reference group. In addition, length gain was monitored monthly as well. A parametric growth curve (PGC) model was used to test for equivalence of weight gain in the different treatment arms. In addition to the PGC (parametric growth curve) model, GLM (generalized linear model (ANCOVA)) and AMN (Arbitrary Means Model) models were used in order check to correctness of the model. ANCOVA and AMN generated results similar to the PGC, indicating the validity of the PGC model.

### Results

Weight gain per day (grams/day) was calculated as the "difference in weight" divided by the "difference in elapsed time" to test for superiority. Table 1 shows the weight gain (g/day) over the whole study period (from baseline to week 17). The comparisons to breast-fed reference group led to statistically significant differences for the control group 2, as a result of the higher weight gain in the control group, (p-value control vs. breast-fed 0.028). There was no statistically significant difference over time for infants fed with experimental formula 1 compared to the infants fed with control formula 2, or to the breast-fed reference group. Results of the ITT population were in line with the outcome of the PP population.

**Table 1a: Infants' weight gain (g/day) from baseline to Week 17, (PP)**

| | | | | P-value¹ | | |
|---|---|---|---|---|---|---|
| | Experimental Formula [N=71] | Control Formula [N=74] | Breast-fed reference [N=86] | Experime ntal vs. Control | Experime ntal vs. Breast-fed | Control vs. Breast-fed |
| n (Nmiss) | 55 (4) | 58 (7) | 60 (0) | | | |
| Mean (SD) | 28.31 (7.38) | 30.14 (6.56) | 27.66 (5.55) | 0.166 | 0.593 | 0.028 |
| 95% C.I. | 26.32 - 30.31 | 28.42 - 31.87 | 26.22 - 29.09 | | | |
| Median (Q1-Q3) | 27.7 (22.7 - 32.1) | 29.3 (26.1 - 33.8) | 27.3 24.3 - 30.7) | | | |
| Min - Max | 13.3 - 54.3 | 17.5 - 48.7 | 15.9-46.1 | | | |

| | | | | | | |
|---|---|---|---|---|---|---|
| [N]=Number of subjects of the analysis population, [n]=number of non-missing subjects, [Nmiss]=number of missing subjects. ¹: Two sample t-test. Values outside 10-day visit windows are excluded. If age > 135 days, the visit is excluded. [N-n+n-1 drop outs] | | | | | | |

**Table 1b: Infants' weight gain (g/day) from baseline to Week 17, (ITT)**

| | | | | P-value¹ | | |
|---|---|---|---|---|---|---|
| | Experimental Formula [N=94] | Control Formula [N=105] | Breast-fed reference [N=100] | Experime ntal vs. Control | Experime ntal vs. Breast-fed | Control vs. Breast-fed |
| n (Nmiss) | 67 (0) | 78 (1) | 68 (0) | | | |
| Mean (SD) | 28.48 (7.10) | 30.34 (6.56) | 27.36 (5.43) | 0.104 | 0.307 | 0.004 |
| 95% C.I. | 26.75 - 30.21 | 28.86 - 31.82 | 26.05 - 28.68 | | | |
| Median (Q1-Q3) | 28.0 (22.8 - 33.6) | 29.7 (26.1 - 34.1) | 26.9 (24.1 - 30.3) | | | |
| Min - Max | 13.3 - 54.3 | 17.5 - 48.7 | 15.9-46.1 | | | |

| | | | | | | |
|---|---|---|---|---|---|---|
| [N]=Number of subjects of the analysis population, [n]=number of non-missing subjects, [Nmiss]=number of missing subjects. ¹: Two sample t-test. Values outside 10-day visit windows are excluded. If age > 135 days, the visit is excluded. [N-n+n-1 drop outs] | | | | | | |

In Table 2 results of the PGC model with contrast estimates for the difference in weight gain (grams/day) between study groups are presented for the PP population. Equivalence in weight gain was confirmed between the experimental group 1 and the breast-fed reference group (CI: -0.5161 to 3.0293 and estimate 1.2566) (p=0.2432), but for the control group 2 no equivalence to breast-fed was demonstrated (CI: 0.7619 to 4.0966 and estimate 2.4292) (p=0.0168), with a higher weight gain in the control group 1. When comparing the 2 formula groups, the 90% confidence intervals of the estimated difference (CI: -2.9260 to 0.6442 and estimate -1.1409) were entire between -0.5*SD and +0.5*SD (equivalence margin), meaning that all comparisons confirmed equivalence in weight gain (g/day) between the experimental group 1 and control group 2 (p=0.2926). Results of the ITT population were in line with the outcome of the PP population.

**Table 2: Contrast estimates for the difference in weight gain (grams/day) between study groups, PP and ITT**

| | | | | | 90% CI | |
|---|---|---|---|---|---|---|
| Comparison PP | Estimate | Std. Error | t Value | P-value | Lower CL | Upper CL |
| Experimental vs. Control | -1.1409 | 1.0823 | -1.05 | 0.2926 | -2.9260 | 0.6442 |
| Experimental vs. Breast-fed | 1.2566 | 1.0747 | 1.17 | 0.2432 | -0.5161 | 3.0293 |
| Control vs. Breast-fed | 2.4292 | 1.0109 | 2.40 | 0.0168 | 0.7619 | 4.0966 |
| Comparison ITT | | | | | | |
| Experimental vs. Control | -1.6712 | 1.0121 | -1.65 | 0.0995 | -3.3397 | -0.0026 |
| Experimental vs. Breast-fed | 0.8150 | 0.9985 | 0.82 | 0.4149 | -0.8314 | 2.4614 |
| Control vs. Breast-fed | 2.4870 | 0.9401 | 2.65 | 0.0085 | 0.9371 | 4.0369 |

*The analysis was performed with the use of a mixed model quadratic curve (PGC) describing weight over time with subject intercept and subject slope as random factors. Sites within the same country with less than 5 subjects were merged. Site effect was taken into account as a fixed effect. Treatment, sex and age were taken as fixed factors. The covariance structure was taken as "unstructured".*

No statistically significant difference in length gain was observed between experimental formula group 1, control formula group 2 and the breast-fed reference group, for the PP group as well as the ITT group.

When analyzing the body mass index (BMI) gain per day (kg/m²/day) it was calculated as the "difference in BMI" divided by the "difference in elapsed time" to test for superiority. Table 3 shows the BMI gain (kg/m2/day) over the whole study period (from baseline to week 17). The comparisons to breast-fed reference group led to statistically significant differences for the control group 2, as a result of the higher BMI gain in the control group, (p-value control vs. breast-fed 0.007). There was no statistically significant difference over time for infants fed with experimental formula 1 compared to the infants fed with control formula 2, or with the breast-fed reference group. Results of the ITT population were in line with the outcome of the PP population, but additionally in experimental group 1 the reduced BMI gain compared with the control formula group 2 was statistically significant (p= 0.012).

**Table 3a: Infants' BMI gain (kg/m²/day) from baseline to Week 17, (PP)**

| | | | | P-value¹ | | |
|---|---|---|---|---|---|---|
| | Experimental Formula [N=71] | Control Formula [N=74] | Breast-fed reference [N=86] | Experi mental vs. Contro 1 | Experim ental vs. Breast-fed | Control vs. Breast-fed |
| n (Nmiss) | 55 (4) | 58 (7) | 60 (0) | | | |
| Mean (SD) | 0.0290 (0.0179) | 0.0342 (0.0155) | 0.0262 (0.0157) | 0.104 | 0.374 | 0.007 |
| 95% C.I. | 0.0242 - 0.0339 | 0.0301 - 0.0383 | 0.0221 - 0.0303 | | | |
| Median (Q1-Q3) | 0.0291 (0.019-0.040) | 0.0323 (0.023-0.043) | 0.0276 (0.015-0.035) | | | |
| Min - Max | -0.019-0.067 | 0.009-0.079 | -0.020-0.089 | | | |

| | | | | | | |
|---|---|---|---|---|---|---|
| [N]=Number of subjects of the analysis population, [n]=number of non-missing subjects, [Nmiss]-number of missing subjects. ¹: Two sample t-test. Values outside 10-day visit windows are excluded. For visit 1 subjects aged > 35 days are excluded. If age > 135 days, the visit is excluded | | | | | | |

**Table 3b: Infants' BMI gain (kg/m²/day) from baseline to Week 17, (ITT)**

| | | | | P-value¹ | | |
|---|---|---|---|---|---|---|
| | Experimental Formula [N=94] | Control Formula [N=105] | Breast-fed reference [N=100] | Experi mental vs. Contro 1 | Experim ental vs. Breast-fed | Control vs. Breast-fed |
| n (Nmiss) | 67 (0) | 78 (1) | 87 (1) | | | |
| Mean (SD) | 0.0290 (0.0171) | 0.0360 (0.0161) | 0.0260 (0.0150) | 0.012 | 0.283 | <0.01 |
| 95% C.I. | 0.0249 - 0.0332 | 0.0324 - 0.0397 | 0.0224 - 0.0297 | | | |
| Median (Q1-Q3) | 0.0294 (0.020-0.038) | 0.0329 (0.026-0.047) | 0.0276 (0.016-0.035) | | | |
| Min - Max | -0.019-0.067 | 0.0089-0.079 | -0.020-0.089 | | | |

| | | | | | | |
|---|---|---|---|---|---|---|
| N]=Number of subjects of the analysis population, [n]=number of non-missing subjects, [Nmiss]-number of missing subjects. ¹: Two sample t-test. Values outside 10-day visit windows are excluded. For visit 1 subjects aged > 35 days are excluded. If age > 135 days, the visit is excluded | | | | | | |

In Table 4 results of the PGC model with contrast estimates for the difference in BMI gain (kg/m²/day) between study groups are presented for the PP-G population. Equivalence in BMI gain was confirmed between the experimental group 1 and the breast-fed reference group (CI: -0.0007 to 0.0086 and estimate 0.0039) (p=0.1639). For the control group 2 no equivalence to breast-fed was demonstrated (CI: 0.0012 to 0.0097 and estimate 0.0054) (p=0.0334). When comparing the two formula groups the 90% confidence intervals of the estimated difference (CI: -0.0062 to 0.0028 and estimate -0.0017) were entire between -0.5*SD and +0.5*SD (equivalence margin), meaning that all comparisons confirmed equivalence in BMI gain between the experimental group 1 and control group 2 (p=0.5292). Results of the ITT population were in line with the outcome of the PP population.

**Table 4: Contrast estimates for the difference in BMI gain (kg/m²/day) between study groups, PP and ITT**

| | | | | | 90% CI | |
|---|---|---|---|---|---|---|
| Comparison PP | Estimat e | Std. Error | t Value | P-value | Lower CL | Upper CL |
| Experimental vs. Control | -0.0017 | 0.0027 | -0.63 | 0.5292 | -0.0062 | 0.0028 |
| Experimental vs. Breast-fed | 0.0039 | 0.0028 | 1.40 | 0.1639 | -0.0007 | 0.0086 |
| Control vs. Breast-fed | 0.0054 | 0.0026 | 2.14 | 0.0334 | 0.0012 | 0.0097 |
| Comparison ITT | | | | | | |
| Experimental vs. Control | -0.0044 | 0.0025 | -0.178 | 0.0763 | -0.0085 | -0.0003 |
| Experimental vs. Breast-fed | 0.0024 | 0.0026 | 0.94 | 0.3467 | -0.0018 | 0.0066 |
| Control vs. Breast-fed | 0.0076 | 0.0024 | 2.83 | 0.0049 | 0.0028 | 0.0106 |

*The analysis was performed with the use of a mixed model quadratic curve (PGC) describing weight over time with subject intercept and subject slope as random factors. Sites within the same country with less than 5 subjects were merged. Site effect was taken into account as a fixed effect. Treatment, sex and age were taken as fixed factors. The covariance structure was taken as "unstructured".*

The formula without fermented ingredient, having a protein content of 1.8 g/100 kcal, which is considered to be low in the art, does not show an improved growth trajectory comparable to the breast-fed reference group as such, but in the presence of fermented ingredient the growth trajectory is improved. These results are indicative for a growth trajectory and a body composition development in infants consuming infant formula that is partly fermented that is more similar to the growth trajectory and a body composition development in breast-fed infants. These results are indicative a reduced risk of becoming overweight or obese in infants consuming infant formula that is at least partly fermented.

### Example 2: Eating behaviour differs in infants receiving experimental formula compared to control formula at week 17

In the clinical study of example 1, baby eating behaviour questionnaires (Llewellyn et al. Appetite 2011, 57:388-96) were filled in by the parents. The Baby Eating Behaviour Questionnaire (BEBQ) is an extensively validated tool which has shown to be able to assess eating behaviour in infants. The BEBQ generates scores on the following scales: food responsiveness, enjoyment of food, satiety responsiveness, slowness in eating and general appetite. The Baby Eating Behaviour Questionnaire (BEBQ) has 18 questions with answers on a 5-point scale (never, rarely, sometimes, often, always). The items are used to compute scores on the following scales: food responsiveness (6 items), enjoyment of food (4 items), satiety responsiveness (3 items), slowness in eating (4 items) and general appetite (1 item).

At the first visit (inclusion visit), as expected, no significant differences were observed between the experimental and control group. However, when the infants were 17 weeks of age significant differences were observed, see Table 5, and also the changes when compared with baseline, see Table 6. Satiety responsiveness was increased and general appetite was decreased in the group receiving the experimental formula compared to the control group, whereas slowness in eating was increased, food responsiveness decreased and enjoyment of food decreased, but did not show significant differences (Wilcoxon-Mann-Whitney tests).
When the infants were 17 weeks of age significant differences in eating behaviour were observed when compared with the baseline. Satiety response was increased, and food responsiveness and general appetite were decreased in the group receiving the experimental formula. Slowness in eating was increased, and enjoyment of food were lower too, but not statistically significant.

**Table 5: BEBQ outcome of infants receiving experimental compared to infants receiving control formula at week 17, ITT**

| Parameter | Experimental Formula [N=94] | Control Formula [N=105] |
|---|---|---|
| n (Nmiss) | 70 (0) | 76 (0) |
| Food responsiveness score Median (Q1-Q3) | 1.8 (1.3-2.3) | 1.8 (1.5-2.7) |
| Enjoyment of food score Median (Q1-Q3) | 4.3 (3.8-4.7) | 4.5 (4.0-4.8) |
| Satiety responsiveness score Median (Q1-Q3) | 2.3 (2.0-3.0)* | 2.0 (1.3-2.7) |
| Slowness in eating score Median (Q1-Q3) | 2.5 (1.8-2.8) | 2.3 (1.5-2.8) |
| General appetite score Median (Q1-Q3) | 4.0 (3.0-4.0)* | 4.0 (4.0-5.0) |

| | | |
|---|---|---|
| Scale: 1=never, 2=rarely, 3=sometimes, 4=often, 5=always. [N]=Number of subjects of the analysis population, [n]=number of non-missing subjects, [Nmiss]=number of missing subjects. *: P < 0.05 compared with control formula at week 17, Wilcoxon-Mann-Whitney test | | |

**Table 6: BEBQ outcome of infants receiving experimental compared to infants receiving control formula at week 17, change from baseline, ITT group**

| Parameter | Experimental Formula [N=94] | Control Formula [N=105] | P-value | Effect Size |
|---|---|---|---|---|
| n (Nmiss) | 70 (0) | 76 (0) | | |
| **Food responsiveness** LS Mean ±SE | -0.1390±0.0869 | 0.1389±0.0840 | 0.0236* | 0.177** |
| **Enjoyment of food** LS Mean ±SE | -0.0607±0.0583 | 0.0467±0.0564 | 0.1878* | 0.314** |
| **Satiety responsiveness** LS Mean ±SE | 0.1530±0.0839 | -0.1258±0.0811 | 0.0184* | 0.468** |
| **Slowness in eating** LS Mean ±SE | 0.0972±0.0902 | -0.0738±0.0871 | 0.1758* | 0.327** |
| **General appetite** Median (Q1-Q3) Mean (sd) | 0.0 (-1.0 - 0.0) -0.44 (0.81) | 0.0 (0.0-0.0) 0.04 (0.88) | 0.002# | 0.2515## |

Scale: 1=never, 2=rarely, 3=sometimes, 4=often, 5=always. [N]=Number of subjects of the analysis population, [n]=number of non-missing subjects, [Nmiss]=number of missing subjects, LS Mean: least Square means, SE: standard error, *P-value ANCOVA, #P-value Wilcoxon-Mann-Whitney test, ** Effect size Cohen's D, ## Effect size Cramers V

As shown in Llewellyn et al. (Appetite 2011, 57:388-96), Mallan et al. (Appetite, 2014; 82:43-49) and Jaarsveld et al. (JAMA Pediatr 2014, 168(4): 345-350) all the observed changes on eating behaviour, even if not reaching significance, in the experimental group are indicative for a decreased risk of becoming overweight or obese later in life.

Data on formula intake also suggest a correlation between the eating behaviour, in particular satiety responsiveness and general appetite, and the intake of formula. Without wishing to be bound by theory, the growth trajectories with lower weight gain and BMI gain observed in the experimental group, being closer to the growth trajectories in the breast-fed reference group, could be explained by a better self-regulation of energy intake due to improved eating behaviour.

Both the observed effect on eating behaviour and the growth trajectories being more similar to breast-fed reference group further indicate a decreased risk of becoming overweight or obese later in life for infants consuming a (partly) fermented infant formula.

### Example 3: Early in life growth trajectories and body composition development in infants consuming infant formula comprising partly fermented formula, comprising non-digestible oligosaccharides or comprising both

The following data are shown to demonstrate that the effect on growth trajectory is mainly due to the presence of a partly fermented formula and that the presence of non-digestible oligosaccharides has less effect.
A similar clinical trial as described in example 1 was performed. In a randomized, multi-centre, double-blinded, prospective clinical trial, infants were enrolled before 28 days of age and assigned to 3 test groups to receive one of three formulas:
Test group 1: Infant formula 1 partly fermented with non-digestible oligosaccharides (50F+); infant formula 1 comprising per 100 ml 66 kcal, 1.35 g protein (bovine whey protein/casein in 1/1 weight ratio), 8.2 g digestible carbohydrate (of which 5.6 g lactose, and 2.1 g maltodextrin), 3.0 g fat (mainly vegetable fat), 0.8 g non-digestible oligosaccharides comprising scGOS (source Vivinal® GOS) and lcFOS (source RaftilinHP®) in a 9:1 wt. ratio. Of this infant formula about 50 wt% based on dry weight is derived from Lactofidus™. The infant formula comprises about 0.55 wt% lactic acid + lactate based on dry weight, of which at least 95% is L(+)-lactic acid/lactate. The composition further comprises vitamins, minerals, trace elements and other micronutrients according to international directive 2006/141/EC for infant formula.
Test group 2: Infant formula 2 partly fermented without non-digestible oligosaccharides (50F-); similar to infant formula 1, but without the non-digestible oligosaccharides scGOS and lcFOS. Test group 3: Infant formula 3 (0F+); a non fermented infant formula with 0.8 g non-digestible oligosaccharides comprising scGOS (source Vivinal® GOS) and lcFOS (source RaftilinHP®) in a 9:1 wt ratio, and for the remainder similar in composition as infant formula 1.

In the statistical analysis comparisons of interest have been investigated. The effect of fermented formula was assessed by comparing test group 3 (0F+) and 1 (50F+). The effect of non digestible oligosaccharide addition was assessed by comparing test group 3 (50F+) and 2 (50F).

From the total of 432 randomized subjects 16 subjects were excluded from the All-Subjects-Treated (AST) population since they did not consume any study product. One subject was diagnosed with hypothyroidism and was considered as not healthy, erroneously randomized and excluded from ITT analysis. A total of 276 subjects completed the study until 17 weeks of age, whereas 155 (36%) subjects dropped-out from the study prematurely. The number of subjects that completed the study was not apparently different between groups. There were no differences in reasons for drop-out between groups. The Per Protocol population was 79, 65, and 74 subjects for group 1, 2 and 3, respectively.
At baseline, no differences in subject characteristics, including sex ratio, of the PP intervention groups were observed, except for a higher number of first-born infants in group 1 versus group 2, which is considered to be statistical significant different by chance and without clinical relevance. Baseline anthropometric measures were not statistically different between treatment groups.

### Results

The mean weight gain (gram/day) in the PP population at week 17 was highest in the test group 3 with non-digestible oligosaccharides but without fermented ingredient and lowest in the test group 2 with fermented ingredient, but without non digestible oligosaccharides. The median weight gain showed a similar pattern. The mean length gain (mm/day) on the other hand was quite similar in group 2 and 3, but higher in group 1, see Table 7.

**Table 7: The gain in weight (gram/day) and in length (mm/day) per formula from study entry to 17 weeks of age for the Per Protocol group.**

| | | Test group 1 50F+ | Test group 2 50F- | Test group 3 0F+ |
|---|---|---|---|---|
| Weight gain g/day | Mean | 28.727 | 28.235 | 29.734 |
| | Median | 28.12 | 27.89 | 29.42 |
| Lengthgain mm/day | Mean | 1.135 | 1.092 | 1.078 |
| | Median | 1.15 | 1.09 | 1.10 |

Using PGC (parametric growth curve) analysis similar as in example 1, the estimated difference in weight gain in group 3 (0F+) versus group 1 (50F+) was 0.15 and the length gain was -0.05. In other words in the group consuming the formula with the fermented ingredient, the weight gain was lower than in the group consuming a formula with no fermented ingredient whereas the length gain in both groups was similar. In both formula non digestible oligosaccharides were present.

Likewise the estimated difference in weight gain in group 2 (50F vs. 50F+) versus group 1 was -0.31 and the length gain was -0.03. In other words in the group consuming the formula with the fermented ingredient but without non digestible oligosaccharides, the weight gain was lower than in the group consuming a formula with fermented ingredient with non-digestible oligosaccharides and the length gain in both groups was similar.
In the same study a group receiving the same formula as test group 1 with the only difference that instead of 50 wt% it contained 15 wt% Lactofidus™ based on dry weight of the composition. The results on growth trajectory for the 15 wt% fermented ingredient were even more advantageous than the 50 wt% showing the beneficial effect over a broad range.

These effects on weight and length gain are indicative for a decreased weight for length gain and a decreased BMI gain being mainly due to consuming partly fermented formula and not, or to a lesser degree, due to consuming formula with non-digestible oligosaccharides. These results show that the beneficial effect on growth trajectory and body development are mainly caused by the presence of a partly fermented infant formula, and not by the presence of non-digestible oligosaccharides. In this clinical study no breast-fed reference group or control group without fermented ingredient and without non digestible oligosaccharides were included, so only conclusions can be drawn on the effect of fermented ingredient lowers weight gain and lowers BMI gain, whereas the presence of non-digestible oligosaccharides has no or a less effect.

## Claims

1. A nutritional composition selected from an infant formula and a follow on formula comprising 3 to 7 g lipid/100 kcal, 1.25 to 5 g protein/100 kcal and 6 to 18 g digestible carbohydrate/100 kcal and comprising an ingredient fermented by lactic acid producing bacteria and 0.05 to 1.5 wt% lactic acid based on dry weight, for use in reducing the risk of overweight and/or obesity in an infant and/or preventing overweight and/or obesity in an infant.

2. The nutritional composition for use according to claim 1, wherein the nutritional composition comprises 1.6 to 2.1 g protein/100 kcal.

3. The nutritional composition for use according to claim 1 or 2, wherein the nutritional composition comprises 0.25 wt% to 20 wt% of non-digestible oligosaccharides based on dry weight of the nutritional composition.

4. The nutritional composition for use according to claim 3, wherein the non-digestible oligosaccharides are selected from the group consisting of fructo-oligosaccharides and galacto-oligosaccharides.

5. The nutritional composition for use according to any one of the preceding claims, wherein the lactic acid producing bacteria are *Bifidobacterium breve* and/or *Streptococcus thermophilus.*

6. The nutritional composition for use according to any one of the preceding claims, wherein the nutritional composition comprises inactivated lactic acid producing bacteria.

7. The nutritional composition for use according to any one of the preceding claims, wherein the nutritional composition is an infant formula.

8. The nutritional composition for use according to any one of the preceding claims, wherein the nutritional composition comprises 1.7 to 2.0 g protein/100 kcal.

9. The nutritional composition for use according to any one of the preceding claims, wherein the nutritional composition comprises 1.75 to 1.95 g protein/100 kcal.

10. The nutritional composition for use according to any one of the preceding claims, wherein the nutritional composition comprises lactic acid and lactate in an amount of 0.1 to 0.75 wt% based on dry weight of the composition.

11. The nutritional composition for use according to any one of the preceding claims, wherein the nutritional composition comprises n-3 long chain polyunsaturated fatty acids (LC-PUFA) in an amount that does not exceed 15 wt.% of the total fatty acid content, and that is at least 0.15 wt.% based on fatty acid content.

12. The nutritional composition for use according to any one of the preceding claims, wherein the infant is exposed to an obesogenic environment and/or a Western type diet later in life.
